# EUROPEAN PATENT SPECIFICATION

(11) **EP 3 260 124 B1**
(45) Date of publication and mention of the grant of the patent: **12.07.2023**
(21) Application number: 17181203.5
(22) Date of filing: 14.05.2012
(51) Int. Cl.: A61K 38/38, A61K 31/727, A61K 35/16, A61K 38/57, A61P 1/00, A61P 7/02, A61P 9/06, A61P 9/12, A61P 11/08

(54) **METHODS TO REDUCE ADVERSE EVENTS CAUSED BY PHARMACEUTICAL PREPARATIONS COMPRISING PLASMA DERIVED PROTEINS**
VERFAHREN ZUR VERRINGERUNG VON DURCH PHARMAZEUTISCHE PRÄPARATE MIT PLASMA-ABGELEITETEN PROTEINEN VERURSACHTE UNTERWÜNSCHTE EREIGNISSE
PROCÉDÉ DE RÉDUCTION DES ÉVÉNEMENTS INDÉSIRABLES CAUSÉS PAR LES PRÉPARATIONS PHARMACEUTIQUES COMPORTANT DES PROTÉINES DÉRIVÉES DE PLASMA

(30) Priority: 12.05.2011 EP 11165869; 17.05.2011 US 201161487205 P
(43) Date of publication of application: 27.12.2017
(62) Divisional of application: 12719993.3
(73) Proprietor: CSL Behring GmbH, 35041 Marburg (DE)
(72) Inventor: Schulte, Stefan, 35043 Marburg (DE); Kalina, Uwe, 35041 Marburg (DE); Moses, Michael, 61279 Graevenwiesbach (DE); Feussner, Annette, 35043 Marburg (DE)
(74) Representative: Carpmaels & Ransford LLP

(56) References cited:
- EP-B1- 0 703 922
- US-A- 4 388 232
- US-A1- 2006 035 828
- HOLMER E ET AL: "The molecular-weight dependence of the rate-enhancing effect of heparin on the inhibition of thrombin, factor Xa, factor IXa, factor XIa, factor XIIa and kallikrein by antithrombin", 1981, BIOCHEMICAL JOURNAL 1981 GB, VOL. 193, NR. 2, PAGE(S) 395 - 400, XP002661553, ISSN: 0264-6021 * See abstract and results: heparin potentiates the inhibiting activity of antithrombin (antithrombin III) on clotting factors, including kallikrein *
- OLSON S T ET AL: "High molecular weight kininogen potentiates the heparin-accelerated inhibition of plasma kallikrein by antithrombin: role for antithrombin in the regulation of kallikrein.", 16 November 1993 (1993-11-16), BIOCHEMISTRY 16 NOV 1993 LNKD- PUBMED:7692967, VOL. 32, NR. 45, PAGE(S) 12136 - 12147, XP002661554, ISSN: 0006-2960 * See abstract: heparin accelerates the inhibiting activity of antithrombin on kallikrenin *
- MACLENNAN ET AL: "Risks and side effects of therapy with plasma and plasma fractions", BEST PRACTICE & RESEARCH CLINICAL HAEMATOLOGY, BAILLIÈRE TINDALL, vol. 19, no. 1, 1 March 2006 (2006-03-01), pages 169-189, XP005219068, ISSN: 1521-6926
- LEBING W R ET AL: "A HIGHLY PURIFIED ANTITHROMBIN III CONCENTRATE PREPARED FROM HUMAN PLASMA FRACTION IV-1 BY AFFINITY CHROMATOGRAPHY", VOX SANGUINIS, S. KARGER AG, BASEL, CH, vol. 67, no. 2, 1 August 1994 (1994-08-01) , pages 117-124, XP000566540, ISSN: 0042-9007
- BURNOUF T ET AL: "Affinity chromatography in the industrial purification of plasma proteins for therapeutic use", JOURNAL OF BIOCHEMICAL AND BIOPHYSICAL METHODS, AMSTERDAM, NL, vol. 49, no. 1-3, 30 October 2001 (2001-10-30), pages 575-586, XP002353092, ISSN: 0165-022X, DOI: 10.1016/S0165-022X(01)00221-4

## Description

The disclosure provides a method to reduce adverse events caused by a pharmaceutical preparation derived from a plasma fraction wherein the method comprises contacting the plasma fraction with heparin or a heparin-like substance thereby reducing the activity of at least one activated serine protease per ml of the plasma fraction.

In the classical waterfall model, blood coagulation proceeds by a series of reactions involving the activation of zymogens by limited proteolysis culminating in the generation of thrombin, which converts plasma fibrinogen to fibrin and activates platelets. In turn, collagen- or fibrin-adherent platelets facilitate thrombin generation by several orders of magnitude via exposing procoagulant phospholipids (mainly phosphatidyl serine) on their outer surface, which propagates assembly and activation of coagulation protease complexes and by direct interaction between platelet receptors and coagulation factors.

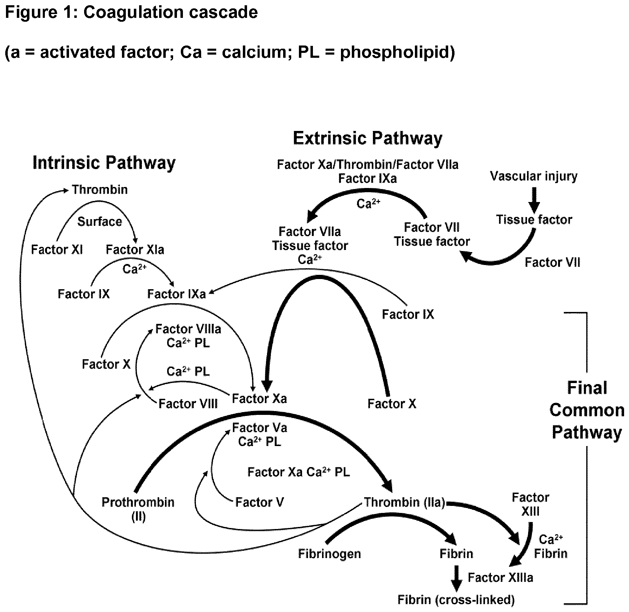

Two converging pathways for coagulation exist that are triggered by either extrinsic (vessel wall) or intrinsic (blood-borne) components of the vascular system (Figure 1). The "extrinsic" pathway is initiated by the complex of the serine protease factor VII (FVII) with the integral membrane protein tissue factor (TF), an essential coagulation cofactor that is absent on the luminal surface but strongly expressed in subendothelial layers of the vessel and which is accessible or liberated via tissue injury. TF expressed in circulating microvesicles might also contribute to thrombus propagation by sustaining thrombin generation on the surface of activated platelets.

The "intrinsic" or "contact activation pathway" is initiated when the serine protease factor XII (FXII, Hageman factor) comes into contact with negatively charged surfaces in a reaction involving high molecular weight kininogen and the serine protease plasma kallikrein (contact activation). FXII can be activated by macromolecular constituents of the subendothelial matrix such as glycosaminoglycans and collagens, sulfatides, nucleotides and other soluble polyanions or non-physiological material such as glass or polymers. One of the most potent contact activators is kaolin and this reaction serves as the mechanistic basis for the major clinical clotting test, the activated partial thromboplastin time (aPTT), which measures the coagulation function of the "intrinsic" pathway. In reactions propagated by platelets, activated FXII then activates the serine protease FXI to FXla and subsequently FXla activates the serine protease FIX to FIXa. The complex of FVllla, which FVllla has been previously activated by traces of FXa and/or thrombin, and FIXa (the tenase complex) subsequently activates the serine protease FX to FXa which in turn with FVa activates the serine protease prothrombin to thrombin.

Factor Xlla has a number of target proteins, including plasma prekallikrein and factor XI. Active plasma kallikrein further activates factor XII, leading to an amplification of contact activation. Contact activation is a surface mediated process responsible in part for the regulation of thrombosis and inflammation, and is mediated, at least in part, by fibrinolytic-, complement-, kininogen/kinin-, and other humoral and cellular pathways. The inactive precursor of plasma kallikrein, prekallikrein is synthesized in the liver as a one chain a-globulin with a molecular weight of approximately 88 kilodalton (kDa) [3]. Prekallikrein circulates in plasma as a 1:1 complex with HMWK in the concentration of 35-50 µg/mL. The kallikrein is formed by the cleavage of prekallikrein into two chains which are held together by one disulfide bridge. The activation of prekallikrein to kallikrein is brought about by the active FXII (FXlla). The active plasma kallikrein cleaves from the HMWK the biologically very active peptide bradykinin which produces heavy blood pressure decrease, increase of vessel permeability, release of tissue plasminogen activator (t-PA) and mobilization of arachidonic acid. Through these mechanisms the kallikrein-kinin-system influences regulation of the blood pressure, the function of kidney and heart as well as the pathological processes of inflammation (for review, Coleman, R. Contact Activation Pathway, pages 103-122 in Hemostasis and Thrombosis, Lippincott Williams Wilkins 2001; Schmaier A. H. Contact Activation, pages 105-128 in Thrombosis and Hemorrhage, 1998).

In pathological conditions, the coagulation cascade may be activated inappropriately which then results in the formation of hemostatically acting plugs inside the blood vessels. Thereby, vessels can be occluded and the blood supply to distal organs is limited. Furthermore, formed thrombin can detach and embolize into other parts of the body, there leading to ischemic occlusion. This process is known as thromboembolism and is associated with high mortality.

Activated proteases originating from blood plasma proteins may contaminate pharmaceutical preparations of proteins derived from human blood plasma and may be the cause of thromboembolic adverse events (TAEs). Suppliers of plasma derived pharmaceuticals therefore need to ensure that their products do not cause such TAEs which have also been associated with the use of an intravenous immunoglobulin (IVIG) preparation recently. Some authors attribute activated coagulation Factor XI (FXla) with a relevant role in the thrombogenic potential of IVIGs (Alving BM, Tankersley DL, Mason BL, Rossi F, Aronson DL, Finlayson JS. Contact-activated factors: contaminants of immunoglobulins preparations with coagulant and vasoactive properties. J Lab Clin Med1980; 96(2): 334-46).

Apart from thrombotic events like stroke other adverse events may be caused by plasma protein preparation comprising enhanced concentrations of kallikrein FXla or FXlla such as skin reactions, bronchospasms, hypoxia, severe rigors, tachycardia, stomach aches and raised blood pressure.

The development of pure and safe preparations is a major goal of plasma derivative manufacturers, including diminishing or virtually eliminating the risk of IVIG-associated TAEs. Marzo et al. reported that pasteurization may be one means to reduce activated proteases in immunoglobulin preparations (Jose M, Marzo N, Bono M, Carretero M, Maite L, Ristol P, Jorquera J. Pasteurization Inactivates Clotting Enzymes During Flebogamma® And Flebogamma® Dif Production. WebmedCentral IMMUNO-THERAPY 2010;1(12): WMC001425).

In 2010 an i.v. immunoglobulin product was withdrawn due to thromboembolic events (European Medicines Agency. Questions and answers on the suspension of the marketing authorisations for Octagam (human normal immunoglobulin 5% and 10%). Outcome of a procedure under Article 107 of Directive 2001/83/EC. 23 September 2010) which led to the suspension of the marketing authorization of the respective product.

US4388232 describes a method for reducing side-effects caused by a plasma fraction comprising antithrombin III. US 2006/035828 describes a method for activating antithrombin III in plasma fractions by the addition of heparin. Homer E et al (Biochemical Journal 193(2): 395-400 (1981)) describes the molecular-weight dependence of the rate-enhancing effect of heparin on the inhibition of thrombin, factor Xa, factor IXa, factor Xlla and kallikrein by antithrombin. Olson S et al (Biochemistry 32(45): 12136-12147 (1993)) describes how high molecular weight kininogen potentiates the heparin-accelerated inhibition of plasma kallikrein by antithrombin and the role for antithrombin in the regulation of kallikrein. Maclennan et al (Best Practice & Research Clinical Hematology 19(1): 169-189 (2006)) describes risks and side effects of therapy with plasma and plasma fractions. Lebing W R et al (Vox Sanguins 67(2): 117-124 (1994)) describes a highly purified antithrombin III concentrate prepared from human plasma fraction IV-1 by affinity chromatography.

There is a clear medical need to develop alternative methods which can be used to improve the safety of plasma derived pharmaceutical preparations.

The present invention provides a solution to this problem. It was found that an adsorption of a pharmaceutical preparation or its intermediate fraction derived from plasma to heparin or heparin-like matrices can substantially reduce the amount of activated proteases and can thus considerably improve the safety of said pharmaceutical preparation.

A method to reduce adverse events caused by a pharmaceutical preparation derived from a plasma fraction wherein the method comprises contacting the plasma fraction with heparin or a heparin-like substance covalently bound to a matrix thereby reducing the activity of at least one activated serine protease per ml of the plasma fraction is disclosed in a first instance.

In a second instance a method to reduce adverse events caused by a pharmaceutical preparation derived from a plasma fraction wherein the plasma fraction has been preadsorbed to an anion exchange (AEX) matrix and the method comprises contacting the plasma fraction with heparin or a heparin-like substance thereby reducing the activity of at least one activated serine protease per ml of the plasma fraction is disclosed.

Preferably in the methods of the first and second instances of the disclosure the plasma fraction comprises antithrombin III (AT III).

The invention provides a method to reduce FXla activity in a pharmaceutical preparation derived from a plasma fraction comprising antithrombin III wherein the plasma fraction has been preadsorbed to an anion exchange (AEX) matrix and the method comprises contacting the plasma fraction with heparin thereby reducing the activity of FXla per ml of the plasma fraction, wherein the heparin which is brought into contact with the plasma fraction is covalently bound to a matrix, and wherein the plasma fraction is an 8% ethanol supernatant I.

The contacting of plasma fractions such as a plasma fraction comprising ATIII with heparin or a heparin-like substance particularly when covalently bound to a matrix (eg. heparin affinity resin) provides a general method to remove activated serine proteases from plasma fractions. Thus where new or adapted plasma fractions are introduced as part of a manufacturing process to purify a plasma protein and it is found that activated serine proteases are formed then the methods of the invention and as disclosed herein can be applied to remove these activated serine proteases.

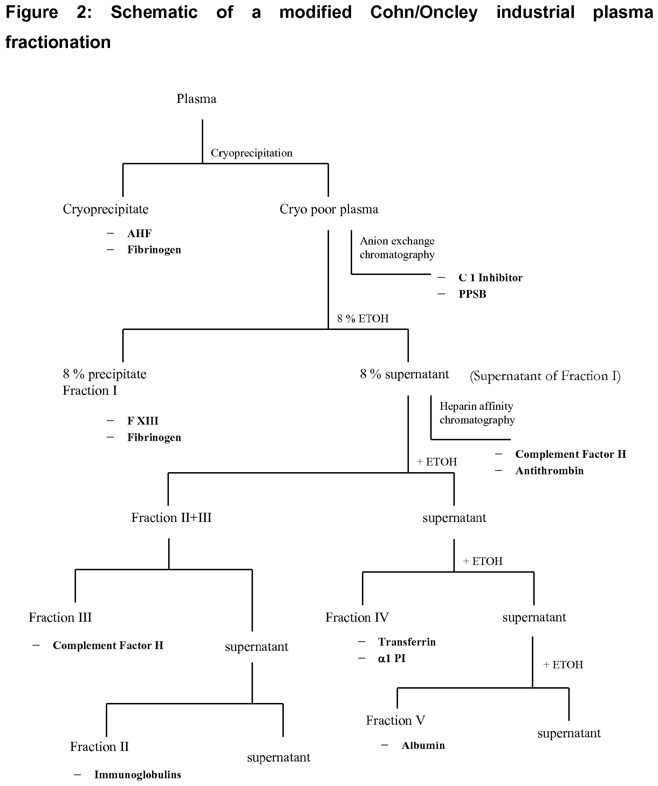

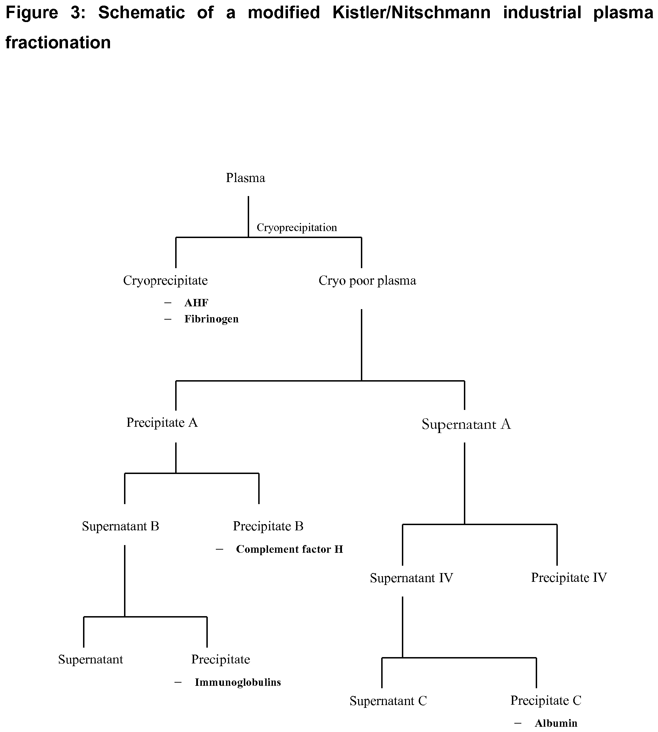

Preferably in the first and second instances of the disclosure the plasma fraction is obtained from a Cohn/Oncley or Kistler/Nitschmann industrial plasma fractionation. Particular examples of these fractionation processes are described in Figures 2 and 3. More preferably the plasma fraction is selected from the group consisting of cryo-poor plasma, 8 % precipitate fraction I, 8 % ethanol supernatant I, fraction II+III, supernatant II+III, fraction II, supernatant II, fraction III, supernatant III, fraction IV, supernatant IV, fraction V, supernatant V, precipitate A or supernatant A, precipitate B, supernatant B, precipitate C or supernatant C. Most preferably the plasma fraction is cryo-poor plasma or 8 % ethanol supernatant I. In the methods of the invention, the plasma fraction is 8 % ethanol supernatant I. Moreover, plasma fractions that are part of an immunoglobulin manufacturing process are also preferred.

Surprisingly it has been found that not just negatively charged materials can activate serine proteases but also positively charged materials such as AEX matrices or resins can activate serine proteases like Kallikrein, Factor XI and Factor XII. Thus the manufacturing of pharmaceutical preparations from plasma which involve exposure to positively charged materials provide the potential to activate serine proteases like Kallikrein, Factor XI and Factor XII. Thus in the invention the plasma fraction is pre-adsorbed to an anion exchange (AEX) matrix. Preferably the AEX matrix is either DEAE, QAE or an anion exchange membrane. More preferably the AEX matrix is used to adsorb Prothrombin complex (PT adsorption) and or to adsorb c1-esterase inhibitor (C1 adsorption).

In the method of the invention the AEX matrix preadsorption of the plasma fraction comprises contacting an intermediate of the plasma fraction with the AEX matrix,wherein the plasma fraction is 8 % ethanol supernatant I.

It will be understood that preferably more than about 80%, 85%, 90%, 95%, or 100% of the plasma fraction is contacted to the heparin or a heparin-like substance in either a soluble form or covalently bound to a matrix. This is in contrast to current plasma fractionation methods where ATIII adsorption is an optional step and is often conducted on a relatively small proportion of the total plasma fraction.

The invention involves a plasma fraction that has been preadsorbed to an anion exchange (AEX) matrix and it will be understood that preferably more than about 80%, 85%, 90%, 95%, or 100% of the plasma fraction is contacted to AEX matrix. This is in contrast to current plasma fractionation methods wherein such steps are optional and are often conducted on a relatively small proportion of the total plasma fraction.

Human blood plasma is industrially utilized for decades for the manufacturing of widely established and accepted plasma-protein products such as e.g. human albumin, immunoglobulin preparations (IgG), clotting factor concentrates (clotting Factor VIII, clotting Factor IX, prothrombin complex etc.) and inhibitors (Antithrombin III, C1-inhibitor etc.). In the course of the development of such plasma-derived drugs, plasma fractionation methods have been established, leading to intermediate products enriched in certain proteins, which then serve as the starting material for the according plasma-protein product. Typical processes are reviewed e.g. in Schultze HE, Heremans JF; Molecular Biology of Human Proteins. Volume I: Nature and Metabolism of Extracellular Proteins 1966, Elsevier Publishing Company; p. 236-317 and simplified schematics of such processes are given in Figure 2 (Cohn/Oncley) and Figure 3 (Kistler Nitschmann).

As can be readily seen from Figures 2 and 3 the manufacturing methods involve a series of steps which result in multiple plasma fractions each comprising a different composition of proteins derived from the human blood plasma source. Plasma fractions such as Cryo-poor plasma, 8 % supernatant, Fraction II+III and the like which require further steps to prepare a therapeutic plasma protein are often referred to more generally as intermediate fractions, intermediate supernatants, intermediate products, intermediates or similar. Plasma fractions at the end of the fractionation process such as Fraction II (immunoglobulins) and Fraction V (albumin) from Figure 2 that have been sufficiently enriched for the particular plasma derived protein (for example, Albumin) or a particular mixture of proteins (for example, Prothrombin complex (PT)) are then prepared as a pharmaceutical preparation (sometimes referred to as a drug product). This can involve additional steps related to for example formulation and pathogen reduction (See Example 1.2, below).

These kinds of separation technologies allow for the manufacturing of several therapeutic plasma-protein products from the same plasma donor pool being economically advantageous over producing only one plasma-protein product from one donor pool and have, therefore, being adopted as the industrial standard in blood plasma fractionation. Typical donor plasma pools used in industrial scaled manufacturing processes range in plasma volume from about 5000 liters to about 70000 liters.

In a first step FVIII, von Willebrand factor and fibrinogen are precipitated from plasma (cryoprecipitation) and the remaining cryo-poor plasma may be adsorbed to matrices to isolate proteins of the prothrombin complex (PT adsorption, PPSB) and or to adsorb C1-inhibitor (C1 adsorption). Usually this adsorption is done using anion-exchange (AEX) matrices like DEAE or QAE.

In the Cohn process then a precipitation at 8% ethanol is done which precipitates FXIII and more fibrinogen. The 8% ethanol supernatant can be subjected directly to further precipitation steps by increasing the ethanol concentration to make further plasma fractions and ultimately leading to pharmaceutical preparations like immunoglobulins, albumin, complement factor H, transferrin and alpha-I-proteinase inhibitor.

Optionally the 8% supernatant may be additionally adsorbed to isolate antithrombin III (AT III adsorption). This step is usually done by using heparin or heparin-like substances.

Large scale purification of AT III typically involves the use of heparin affinity chromatography using heparin or heparin-like substances linked to a matrix (See for example Burnouf & Radosevich, 2001 J. Biochem. Biophys. Methods 575-586). These matrices are often referred to simply as heparin affinity media or resins. Examples of such heparin affinity resins include Heparin-Agarose, Heparin-Acrylic beads, Heparin-Ceramic HyperD Hydrogel composite, Poros-Heparin and Heparin-Sepharose. Such resins can be either purchased off the shelf or made in-house using resins such as Fractogel which can be coupled to heparin or heparin like substances.

The heparin affinity resins are typically either packed into a column and the plasma fraction passed through the column (see Example 1.2) or alternatively it is added directly to the plasma fraction in batch mode to adsorb AT III. In this later method removal of AT III / heparin affinity resin can be achieved by either centrifugation or filtration. The AT III can then be desorbed from the media and further processing can be conducted to make an AT III pharmaceutical preparation. The AT III depleted plasma fraction can then also be subjected to further steps to prepare other plasma derived proteins such as immunoglobulins and albumin. Importantly the heparin affinity resin adsorption step allows the activated serine proteases to be bound either directly or indirectly via ATIII to the heparin or heparin like substance which can then be removed from the plasma fraction and hence the pharmaceutical preparation.

Current manufacturing processes normally allow some flexibility such that not always all adsorptions are done depending on the relative demand for the different products. In the production of immunoglobulins and albumin there may be either:
1: No adsorption steps performed
2: PT adsorption step is solely performed
3: PT adsorption is followed by adsorption of C1 esterase inhibitor
4: PT adsorption is followed by AT III adsorption
5: Complete adsorption process (adsorption of PT, C1 esterase inhibitor and AT III)

These adsorption steps may be performed on the same plasma fraction (for example PT and C1 adsorption steps can be performed on cryo-poor plasma) or on related intermediate fractions thereof (for example AT III adsorption can be performed on the 8 % ethanol supernatant I plasma fraction where the preceding cryo-poor plasma intermediate had PT and or C1 adsorbed).

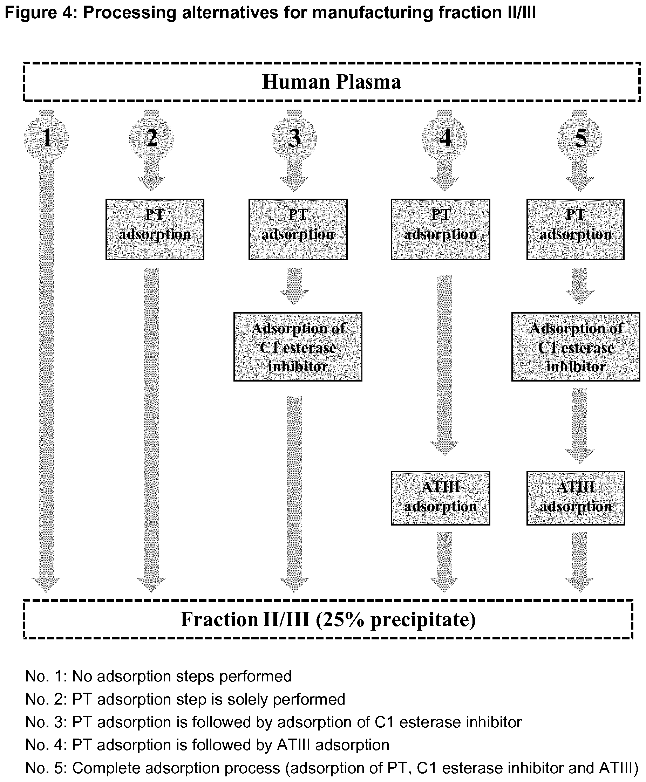

A graph depicting said alternative manufacturing methods is shown in Figure 4. An example of such a manufacturing process is described in Example 1.2.

It has now been found that especially after an adsorption with AEX matrices during the PT and the C1 adsorption activated serine proteases like kallikrein, FXla or FXlla could be detected in subsequent products. Surprisingly pharmaceutical preparations prepared by methods which in addition to an adsorption to an AEX matrix were also adsorbed to heparin or heparin-like substances showed significantly reduced levels of kallikrein and/or FXla-like activity. This leads to a significantly decreased procoagulatory activity of pharmaceutical preparations depleted of AT III. This reduced procoagulatory activity reduces the risk of adverse events when such a product is administered to patients. Examples of adverse events are thromboembolic events, skin reactions, bronchospasms, hypoxia, severe rigors, tachycardia, stomach aches and raised blood pressure.

Not wanting to be bound by theory this effect may be explained in that the AEX matrices activate FXII to FXIIa which in turn activates prekallikrein to kallikrein and FXI to FXla. A further adsorption to remove C1 inhibitor may lead to further activation and also removes C1 inhibitor an important inhibitor of kallikrein. When these kallikrein FXla and FXlla containing fractions subsequently come into contact with heparin or heparin-like matrices, AT III - which is still usually present at this stage of plasma protein processing - binds to the heparin or heparin-like matrix, is activated and subsequently inactivates FXla and kallikrein by irreversibly binding to both proteins, thereby removing these potential thrombogenic proteins. Therefore the disclosed methods will be applicable in any solution comprising plasma proteins which may contain activated serine proteases as long as the solution also comprises AT III.

AT III is a plasma protein and a serine proteinase inhibitor that inactivates thrombin and the other serine proteases responsible for the generation of thrombin. The anticoagulant activity of heparin or heparin-like substances derives from their ability to potentiate the inhibitory activity of AT III by mechanisms that are similar to the physiologic activation of AT III by vessel wall heparin sulfate proteoglycans (HSPGs). AT III serves as an important regulator of hemostasis and thrombosis at several levels by blocking (a) thrombin-mediated fibrin clot formation, (b) common pathway factor Xa mediated thrombin generation, and (c) coagulation factors that are higher up in the intrinsic and extrinsic pathways (FIXa, FXla, FXlla and plasma kallikrein and FVIIa (Colman et al., Hemostasis and Thrombosis, 5th edition, 2006 Lippincott Williams, p. 235 f.).

Binding of AT III to heparin or heparin-like substance leads to a conformational change in AT III transforming the molecule into a highly active state which has a several thousand fold enhanced inhibitory activity to activated serine proteases like activated coagulation factors by forming irreversibly a covalent bond to the activated serine protease. Upon forming this covalent bond the serine protease loses irreversibly its biological function as a serine protease.

The disclosure is therefore about a method to reduce adverse events caused by a pharmaceutical preparation derived from a plasma fraction said plasma fraction preferably comprising antithrombin III wherein the method of the invention comprises contacting the plasma fraction with heparin thereby reducing the activity of FXla per ml of the plasma fraction.

A "heparin or heparin-like substance" in the sense of the invention is any form of heparin or heparin-related substance which cause when contacting AT III the activation of AT III, i.e. that AT III adapts the conformation which has a high affinity to form covalent complexes with activated serine protease, preferentially activated coagulation factors.

Heparin-like substances consist of a group of products derived from heparin, made by one or more chemical modifications. For example, sulfated heparin is a derivative in which all primary hydroxyls in glucosamine residues and a large proportion of secondary hydroxyl groups in disaccharide units have been substituted by O-sulfate esters; carboxyl reduced heparin is a derivative in which the carboxyl group of uronic acid residues of heparin have been reduced to alcohols; periodate-oxidized heparin is a derivative in which all unsulfated uronic acid residues of heparin are oxidized by periodic acid. Other heparin derivatives include, for example, de-O-sulfated heparin, 2-O-desulfated heparin, fully N-acetylated heparin, fully N-sulfated heparin, de-N-sulfated heparin, de-N-acetylated heparin. "Heparin or heparin-like substances" in the sense of the invention encompass unfractionated heparin, high-molecular weight heparins, low-molecular weight heparins and synthetic heparin analogues like fondaparinux.

"Heparin or heparin-like substances" may be used according to the method of the invention as claimed, the method comprising contacting the plasma fraction as referred to in the claims, with heparin thereby reducing the activity of FXla per ml of the plasma fraction, wherein the heparin which is brought into contact with the plasma fraction is covalently coupled to a matrix. Here the covalent complex of AT III with the activated coagulation factor remains bound to the matrix. This provides a particular advantage in that the activated proteases along with the heparin or heparin like substance covalently bound to a matrix (ie. heparin affinity resin) are then easily removed from the plasma fraction using methods such as centrifugation or filtration. As a consequence there are no on-going problems with for example the possibility of highly charged heparin or heparin like substances being present in the pharmaceutical preparation (Such molecules because of their highly charged nature can be extremely difficult to remove in subsequent fractionation processing steps). A further problem overcome by contacting the plasma fraction with heparin or a heparin-like substance covalently bound to a matrix which is then removed from the plasma fraction is that it prevents the possibility of activated serine proteases being inadvertently reintroduced to the plasma fraction, later intermediates or the pharmaceutical preparation itself due to dissociation of the ATIII-activated protease complex. It is known for example that ATIII complexed to thrombin will dissociate active thrombin over a period of days (For example see, Danielsson and Björk, FEBS Letters, (1980) 119, 2, 241-244). Alternatively the heparin or heparin-like substance may be added to a plasma fraction as a soluble substance. Then the covalent complex of AT III with the activated coagulation factor either precipitates or remains in solution.

"Reducing the specific activity of at least one activated serine protease per ml of the plasma fraction" in the sense of the invention means that the method of the invention leads to a decrease of the activity of at least one serine protease per volume of the plasma fraction which comprises antithrombin III. The reduction of the activity may be due only to the irreversible binding of the activated serine protease to the heparin-activated antithrombin III, when heparin or the heparin-like substance is added in solution whereas the antigen content of the activated serine protease does not change or may also lead to a reduction of the amount of the activated serine protease if the heparin or heparin-like substance is coupled to a matrix which is subsequently separated from the plasma fraction and where the serine protease remains covalently coupled to antithrombin III on the matrix.

An "adverse event" asdisclosed herein is any effect caused by the administration of the pharmaceutical preparation caused by activated serine proteases and may comprise thrombosis, skin reactions, bronchospasms, hypoxia, severe rigors, tachycardia, stomach aches and raised blood pressure.

"Plasma derived proteins" according to the invention comprise any protein which is isolated from human plasma after the 8% ethanol precipitation step according to Cohn or an equivalent step according to other methods for plasma fractionation. In a preferred embodiment "plasma derived proteins" in the sense of the invention mean all proteins which are isolated from human plasma where intermediates thereof have been contacted with an AEX matrix. "Plasma derived proteins" according to the invention comprise for example immunoglobulins, albumin, complement factor H, alpha-I-proteinase inhibitor and transferrin.

A "plasma fraction" according to the invention is any plasma derived solution or re-dissolved precipitate, where at least part of the proteins originate from human plasma.

Factor XI is a coagulation protein and a serine protease produced in the liver and circulates in plasma at approximately 5 µg/ml (30 nM). FXI consists of two identical 80 kDa subunits linked by disulfide bonds. Cleavage of FXI by activated factor XII or thrombin converts each subunit into a two-chain form and generates two active sites per FXla molecule (Bagila FA, Seaman FS, Walsh PN. The apple 1 and 4 domains of factor XI act to synergistically promote the surface-mediated activation of factor XI by factor Xlla. Blood 1995; 85:2078). The activity of FXla is regulated by platelets and by several proteinase inhibitors. Natural substrate for FXla is solely FIX; the only cofactor required for this reaction are calcium ions.

Prekallikrein is a 88 kDa single chain glycoprotein produced in the liver. The plasma concentration of PK is 50 µg/ml (550 nM), approximately 75% of which circulates in complex with high molecular weight kininogen and the remainder as free PK (Hojima Y, Pierce JV, Pisano JJ. Purification and characterization of multiple forms of human plasma prekallikrein. J Biol Chem 1985; 260:400-406). Limited proteolysis of PK by FXlla generates the active serine protease kallikrein (Dela Cadena R, Watchtfogel YT, Colman RW. Hemostasis and Thrombosis, 3rd edition 1994. pp. 219-240 ).

Factor XII (Hageman factor) is a 76 kDa, single chain glycoprotein produced in the liver. In plasma, FXII circulates as a protease zymogen at a concentration of approximately 30 µg/ml (400 nM). Upon vascular injury FXII binds to negatively charged extravascular surfaces which facilitate activation of the zymogen to the active serine protease (Pixley RA, Schapira M, Coleman RW. The regulation of human factor XIIa by plasma proteinase inhibitors. J Biol Chem 1985; 260(3):1723-1729). The activity of FXlla in plasma is regulated predominantly by C1 inhibitor.

An "intermediate" of a pharmaceutical preparation comprising one or more plasma proteins according to the invention is any intermediate fraction during the purification of said one or more plasma proteins and comprises for example any supernatant from a precipitation step during the purification or any eluate of a matrix used for purification of a plasma derived protein.

In the method of the invention the plasma fraction which is contacted with heparin is prior adsorbed to an anion-exchange matrix (AEX matrix). The skilled addressee will understand that the AEX matrix adsorption can be completed on either the plasma fraction itself or an intermediate of the plasma fraction. An example of this would be when the AEX matrix is used to adsorb PT in cryo-poor plasma and the plasma fraction is the subsequent 8 % ethanol supernatant.

In the sense of the invention an "anion exchange matrix" (AEX matrix) refers to a solid phase which is positively charged at the time of protein binding, thus having one or more positively charged ligands attached thereto. Any positively charged ligand attached to a solid phase suitable to form the anionic exchange matrix can be used, such as quaternary amino groups. For example, a ligand can be a quaternary ammonium, such as quaternary alkylamine and quaternary alkyl alkanol amine, or amine, diethylamine, diethylaminopropyl, amino, trimethylammoniumethyl, trimethylbenzyl ammonium, dimethylethanolbenzyl ammonium, and polyamine..

Commercially available anion exchange matrices which are often also referred to as resins include, for example, DEAE cellulose, POROS^{®} PI 20, PI 50, HQ 10, HQ 20, HQ 50, D 50 from Applied Biosystems, MonoQ^{®}, MiniQ, Source^{™} 15Q and 3OQ, Q, DEAE and ANX Sepharose^{®} Fast Flow, Q Sepharose^{®} high Performance, QAE SEPHADEX^{™} and FAST Q SEPHAROSE^{®} from GE Healthcare, WP PEI, WP DEAM, WP QUAT from J.T. Baker, Hydrocell DEAE and Hydrocell QA from Biochrom Labs Inc., UNOsphere^{™} Q, Macro-Prep^{®} DEAE and Macro-Prep^{®} High Q from Biorad, Ceramic HyperD^{®} Q, ceramic HyperD^{®} DEAE, Q HyperZ^{®}, Trisacryl^{®} M and LS DEAE, Spherodex^{®} LS DEAE, QMA Spherosil^{®} LS, QMA Spherosil^{®} M from Pall Technologies, DOWEX^{®} Fine Mesh Strong Base Type I and Type II Anion Matrix and DOWEX^{®} MONOSPHER E 77, weak base anion from Dow Liquid Separations, Matrex Cellufine A200, A500, Q500, and Q800, from Millipore, Fractogel^{®} EMD TMAE3 Fractogel^{®} EMD DEAE and Fractogel^{®} EMD DMAE from EMD, Amberlite^{™} weak and strong anion exchangers type I and II, DOWEX^{®} weak and strong anion exchangers type I and II, Diaion weak and strong anion exchangers type I and II, Duolite^{®} from Sigma- Aldrich, TSK gel^{®} Q and DEAE 5PW and 5PW-HR, Toyopearl^{®} SuperQ-650S, 650M and 650C3 QAE-550C and 650S, DEAE- 650M and 650C from Tosoh, and QA52, DE23, DE32, DE51, DE52, DE53, Express-Ion^{™} D and Express-Ion^{™} Q from Whatman.

The AEX matrix can also be an anion exchange membrane. Commercially available anion exchange membranes include, for example, Sartobind^{®} Q from Sartorius, Mustang^{®} Q from Pall Technologies and Intercept^{™} Q membrane from Millipore.

### FIGURES

- **Figure 1:**: Coagulation cascade.
- **Figure 2:**: Schematic of a modified Cohn/Oncley industrial plasma fractionation.
- **Figure 3:**: Schematic of a modified Kistler/Nitschmann industrial plasma fractionation.
- **Figure 4:**: Processing alternatives for manufacturing to the fraction II/III stage in Cohn/Oncley industrial plasma fractionation schemes.
- **Figure 5:**: Analytical Results (Predicted Response Graph) for coagulation related serine protease activity as a function of the level of removal of either antithrombin III (AT III), c1-esterase inhibitor (C1) or prothrombin complex (PT) from a pharmaceutical preparation, SC Immunoglobulin. The statistical analysis of testing results was performed by using the computer program Cornerstone Version 5.0 (Applied Materials Co.).
- **Figure 6:**: Correlation between Prekallikrein-Ag and Kallikrein-like activity (a); correlation between Factor XI-Ag and Factor XI-like activity (b).

### EXAMPLES

### Example 1:

### Example 1.1 - Introduction

The analysis of levels of kallikrein and FXla in multiple batches of subcutaneous immunoglobulin suggested higher levels related to batches in which a greater proportion of the plasma fraction was subjected to PT and C1-INH adsorption and only a small amount of the plasma fraction was adsorbed to the heparin affinity resin. While the Vitamin-K dependent factors of the clotting system are adsorbed to DEAE-Sepharose, the factors involved in the contact activation system remain in the Ig-fraction. These factors, namely high molecular weight kininogen complexed to prekallikrein or FXI and FXII are known to be activated by negatively charged surfaces (McMillin CR, et al.: The secondary structure of human Hageman factor (factor XII) and its alteration by activating agents. J Clin Invest. 1974; 54, 1312-22). Surprisingly however the batch analysis suggested that positively charged materials such as anion exchange resins (eg. DEAE & QAE resins) could also activate these serine proteases. Thus the steps of preabsorbing a plasma fraction to an anion exchange
(AEX) matrix and then contacting the plasma fraction with heparin or a heparin-like substance particularly where this substance can be subsequently removed from the plasma fraction provides an ideal means to ensure that the resulting pharmaceutical preparations are essentially free of activated coagulation factors such as FXla. To investigate this further the following studies were conducted.

An analytical investigation of an immunoglobulin for subcutaneous administration (SC Immunoglobulin) was performed. Various analytical methods were applied with regard to the potential presence of trace amounts of activated clotting factors and proteolytic activity in the SC Immunoglobulin.

The evaluation of analytical data revealed that the SC Immunoglobulin batches contain levels of procoagulant activity in correlation to applied variations of the adsorption scheme. A comparison of adsorption schemes of individual batches revealed that higher levels of procoagulant activity are correlated to high Prothrombin complex (PT) and low antithrombin (AT III) adsorption levels during the plasma fractionation process steps.

Based on the finding of procoagulant activity the production process was adapted to include maximum AT III adsorption. The subsequent examples provide strong evidence that a high level of AT III adsorption leads to a significant decrease in the procoagulant activity of the SC Immunoglobulin product.

### Example 1.2 - Manufacturing Process for a SC Immunoglobulin

The drug substance was prepared by a modified Cohn Fractionation (Cohn EJ, Strong LE, et al. Preparation and properties of serum and plasma proteins; a system for the separation into fractions of the protein and lipoprotein components of biological tissues and fluids. J Am Chem Soc 1946; 68:459-75). Plasma was thawed, the formed cryoprecipitate was separated and contained fibrinogen and antihemophilic Factor VIII / von Willebrand factor complex. With the supernatant, cryo-depleted plasma (also known as cryo-poor plasma), optional batch adsorption of the prothrombin complex (PT adsorption) and C1 esterase inhibitor (C1 adsorption) could be optionally performed (see Figure 4). Subsequently, ethanol was added to the cryo-depleted plasma or filtrate from previous adsorption(s) to adjust an ethanol concentration of 8%. The precipitate, Cohn Fraction I, mainly contained fibrinogen and factor XIII and was separated by filtration. With the 8 % ethanol (Fraction I) supernatant an optional batch adsorption of antithrombin III could be performed.

60 to 100 mL heparin affinity resin per liter cryo-depleted plasma was suspended with the same quantity of Fraction I supernatant in a chromatography column. The obtained suspension is added to the residual quantity of the batch for fractionation. The pH value is adjusted to 6.5 (±0.1) with hydrochloric acid while stirring. The total stirring time is 45 to 60 min at a product temperature of 0 (±2) °C. Subsequently, the product solution is filtered through a filter bag and the filtrate is transferred for further plasma fractionation.

The Fraction I supernatant or flow through fraction from previous AT III adsorption was precipitated at an ethanol concentration of 25%. The resulting precipitate, Cohn Fraction II/III, was obtained by centrifugation and contained mainly immunoglobulins. Fraction II/III is frozen and stored at -20°C or below.

After dissolution in an aqueous glycine solution the fraction II/III was further precipitated at 10% ethanol concentration in the presence of 0.5% fatty alcohol (also referred to as 10% pre-precipitation because it precedes the main 20% precipitation). The precipitate containing mainly IgM, IgA and lipoproteins was removed by filtration.

The supernatant was further precipitated at an ethanol concentration of 20%. The formed precipitate which consisted mainly of IgG (Gammaglobulin paste) was obtained by filtration. Crude Gammaglobulin paste was frozen. Afterwards, it was dissolved and subjected to adsorption by using an ion exchange resin and activated carbon to remove residual albumin and fatty alcohol. Impurities bound to the resin and activated carbon were removed by filtration, respectively. The filtrate was subsequently stabilized with sucrose and glycine. The stabilized solution was pasteurized as an effective virus reduction step. After completion of pasteurization, the stabilizers were removed by ultrafiltration (dialysis). The solution was then concentrated to obtain the drug substance, the immunoglobulin ultraconcentrate.

After the pooling process of the immunoglobulin ultraconcentrate lots, bulk adjustment was performed and the adjusted bulk solution was then filtered through clarification cartridge filters followed by sterilizing filtration. Immediately after completion of the filling process, vials were automatically stoppered and sealed with crimp caps.

### EXAMPLE 1.2 - ANALYTICAL METHODS

### 1.2.1 Factor Xla-like activity (aPTT approach in FXI depleted plasma)

The activated partial thromboplastin time (aPTT) is a coagulation test that encompasses all steps of the intrinsic pathway of blood coagulation from the activation of the contact phase system to fibrin formation. During the pre-incubation phase of the aPTT assay, Factor XII was activated by negatively charged surfaces (e.g. Pathromtin SL) and activated Factor XI to Factor Xla in the presence of high molecular weight kininogen. The result of this initial step was to produce FXla. The clot measurement phase of the aPTT assay took place after re-calcification during which FXla activated FIX, thus continuing the cascade through FXa to thrombin.

Factor Xi-deficient plasma was applied and the presence of activated coagulation factor XI in the sample especially led to a decrease in the coagulation time. The sample was considered as 'activated' with lower clotting times caused by FXla-like activity in the sample. A longer clotting time indicated a lower pro-coagulant acivity.

Factor Xi-deficient plasma and Pathromtin SL reagent were incubated for 6 minutes at +37°C. Pathromtin SL is a reagent consisting of phospholipid and a surface activator (silicon dioxide particles) used to activate the factors of the intrinsic coagulation system. Subsequently, a sample was added, together with 25 mM CaCl2 solution, which triggers the coagulation process. The time between CaCl2 addition and clot formation was measured. Buffer was used as control sample and as diluent for product sample preparation. The buffer used for FXla testing experiments consisted of purchased imidazole buffer and 1 % human albumin. Factor Xla reference material was used for quantification purposes and the test data were presented as FXla equivalence.

### 1.2.2 Kallikrein-like activity (chromogenic substrate S-2302)

Kallikrein-like activity was estimated by means of the cleavage of the chromogenic substrate H-D-Pro-Phe-Arg-pNA (chromogenic substrate S-2302, Chromogenix Co.) and absorbance measuring of pNA at 405 nm. S-2302 is a chromogenic substrate which mainly reacts with plasma kallikrein, and therefore is used for the determination of kallikrein-like activity.

After addition of the chromogenic substrate solution, the samples were incubated at +37°C for 30 minutes. The active kallikrein in the sample is able to cleave the substrate in a concentration dependent manner. This led to a difference in absorbance (optical density) between the pNA formed and the original substrate which was measured photometrically at 405 nm. Moreover, the evaluation was performed on the basis of a standard curve by applying commercial standard reference material of kallikrein.

### 1.2.3 Proteolytic activitiy (chromogenic substrates)

The colorimetric determination of proteolytic activity in samples was performed by applying chromogenic substrates. After addition of the chromogenic substrate solution, the samples (1:20 diluted) were incubated at +37°C for 30 minutes. Proteolytic activity in the sample is able to cleave the substrate in a concentration dependent manner. The method for the determination of activity is based on the difference in absorbance (optical density) between the pNA formed and the original substrate. The rate of pNA formation, i.e. the increase in absorbance per second at 405 nm, is proportional to the enzymatic activity and was determined.

The following table (Table 1) provides an overview of the substrates applied within this study and the respective specificity.

**Table 1: Overview of chromogenic substrates applied**

| Label (Chromogenix Co.) | Chromogenic substrate mainly for* |
|---|---|
| S-2302 | Kallikrein-like activity |
| S-2366 | Activated protein C, FXla |
| S-2238 | Thrombin |
| S-2765 | FXa |
| S-2251 | Plasmin, streptokinase-activated plasminogen |
| S-2288 | Broad spectrum of serine proteases, several proteases with arginine specificity |

| | |
|---|---|
| * according to Chromogenix Co., Italy | |

### 1.2.4 Factor XI ELISA

Human FXI antigen in SC Immunoglobulin samples was quantitatively determined by using commercially available paired antibodies (sandwich-style ELISA), e.g. supplied by Coachrom Diagnostika Co. A polyclonal antibody to FXI was coated onto wells of a microtitre plate to capture FXI in the sample or in the standard reference solution. Afterwards, a horseradish peroxidase conjugated antibody to FXI (polyclonal) was added to the wells of the microtitre plate. After removal of unbound antibodies by several washing steps, a peroxidase reactive substrate solution was added which leads to a coloration in a concentration dependent manner.

The coloration was formed in proportion to the amount of FXI present in the sample. This reaction was terminated by the addition of acid and is measured photometrically at 450 nm by utilizing BEPII or BEPIII systems (Siemens Co.). Moreover, a standard curve was applied by using standard human plasma (Siemens Co.).

Human FXI was detected as well as human FXla due to the cross-reactivity of both with the polyclonal paired antibodies applied.

### 1.2.5 Prekallikrein ELISA

Human prekallikrein antigen in SC Immunoglobulin samples was quantitatively determined by using commercially available paired antibodies (sandwich-style ELISA) supplied by Affinity Biologicals Co. A polyclonal antibody to prekallikrein is coated onto wells of a microtitre plate to capture prekallikrein in the sample or in the standard reference solution. Afterwards, a horseradish peroxidase conjugated antibody to prekallikrein (polyclonal) was added to the wells of the microtitre plate. After removal of unbound antibodies by several washing steps, a peroxidase reactive substrate solution was added which led to a coloration in a concentration dependent manner.

The coloration was formed in proportion to the amount of prekallikrein present in the sample. This reaction was terminated by the addition of acid and the color produced quantified by photometric measurement at 450 nm. BEPII or BEPIII systems (Siemens Co.) were used for the determination.

Human prekallikrein was detected as well as human kallikrein due to the cross-reactivity of both with the polyclonal paired antibodies applied.

### 1.2.6 Factor XII ELISA

Human FXII antigen in SC Immunoglobulin samples was quantitatively determined by using commercially available paired antibodies (sandwich-style ELISA), e.g. supplied by Kordia Co. The test approach applied is comparable to the determination of FXI and PK by ELISA technology as mentioned above.

### 1.3 Test results

29 lots of SC Immunoglobulin drug product manufactured at CSL Behring Marburg (Germany) were analyzed for procoagulant activity. The lots were chosen on the basis of their adsorption scheme. The listed percentage of the adsorption rate per SC Immunoglobulin lot is the result of mixing various fraction II/III intermediate lots with different adsorption levels.

For example, the total amount (100%) of fraction II/III pastes used for SC Immunoglobulin sample no. 13 was PT adsorbed, whereas 59.8% was also subjected to the C1 esterase inhibitor adsorption step and 1.8% to the AT III adsorption.

Supplementary testing activities and analyses for SC Immunoglobulin with regard to the potential presence of trace amounts of activated clotting factors and proteolytic activity in SC Immunoglobulin drug product were also initiated. For the identification and quantification of residual clotting factors several complementary approaches were performed:
- Trace amounts of FXI and FXla were measured by a modified aPTT test performed with FXI-deficient plasma.
- Kallikrein-like activity was measured by applying the chromogenic substrate S-2302 (Chromogenix Co.) due to being generally supposed as major impurities of immunoglobulin preparations.
- The potential presence of proteolytic activity was investigated by using chromogenic substrates characterizing a wide range of proteases.
- ELISA technology was used for the determination of FXI-, PK- and FXII-antigen, respectively.

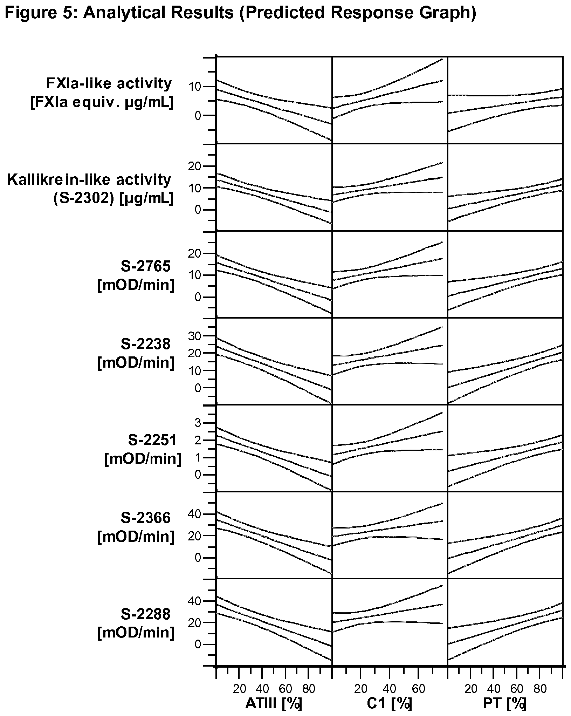

The results of SC Immunoglobulin drug product investigated by FXla-like activity, Kallikrein-like activity and proteolytic activity are summarized in Figure 5. The statistical analysis of testing results was performed using the computer program Cornerstone Version 5.0 (Applied Materials Co.). The evaluation comprises 29 lots of SC Immunoglobulin in total.

**Table 1: Selected lots of SC Immunoglobulin drug product for further analytical evaluation**

| **Sample no.** | **Adsorption scheme [%]** | | | **FXla-like activity** | **Kallikrein-like activity (S-2302) [µg/mL]** |
|---|---|---|---|---|---|
| | **PT** | **C1** | **AT III** | **FXI aequiv. [µg/mL]** | |
| *Lots without any adsorption steps:* | | | | | |
| 1 | 0 | 0 | 0 | 0.06 | < 0.8 |
| 2 | | | | 0.03 | < 0.8 |
| 3 | | | | 0.18 | < 0.8 |
| 4 | | | | 0.08 | < 0.8 |

| *Lots with both 100% PT and AT III adsorption, but differing amounts of C1 adsorbed material:* | | | | | |
|---|---|---|---|---|---|
| 5 | 100 | 41.3 | 100 | < 0.01 | < 0.8 |
| 6 | | 43.4 | | < 0.01 | < 0.8 |
| 7 | | 60.5 | | < 0.01 | < 0.8 |

| *Lots with 100% PT and without almost any AT III adsorption, but differing amounts of C1 adsorbed material:* | | | | | |
|---|---|---|---|---|---|
| 8 | 100 | 0 | 0 | 14.14 | 20.0 |
| 9 | | 8.8 | 0 | 6.56 | 11.9 |
| 10 | | 13.1 | 0 | 11.10 | 15.9 |
| 11 | | 30.8 | 0 | 12.90 | 19.5 |
| 12 | | 34.8 | 0 | 17.96 | 18.8 |
| 13 | | 59.8 | 1.8 | 23.98 | 23.7 |

| *Lots with 100% PT and 70 to 80% C1 adsorbed material, but differing amounts of AT III adsorbed material:* | | | | | |
|---|---|---|---|---|---|
| 14 | 100 | 76.4 | 15.1 | 14.94 | 21.6 |
| 15 | | 72.3 | 59.2 | 3.73 | 12.3 |

| *Additional lots randomly chosen:* | | | | | |
|---|---|---|---|---|---|
| 16 | 41.1 | 41.1 | 63.2 | 0.13 | < 0.8 |
| 17 | 4.6 | 0 | 86.4 | < 0.01 | < 0.8 |
| 18 | 77.7 | 25.0 | 100 | < 0.01 | < 0.8 |
| 19 | 86.5 | 13.5 | 0.3 | 2.13 | 6.0 |
| 20 | 77.4 | 0 | 30.3 | 1.51 | 3.0 |
| 21 | 100 | 6.2 | 55.0 | 0.33 | 5.0 |
| 22 | | 3.3 | 11.9 | 3.42 | 9.8 |
| 23 | | 26.8 | 29.6 | 5.66 | 11.3 |
| 24 | | 14.1 | 58.4 | 2.67 | 7.6 |
| 25 | | 0.9 | 1.8 | 8.11 | 14.4 |
| 26 | | 1.9 | 4.0 | 5.40 | 13.4 |
| 27 | | 30.7 | 40.4 | 8.19 | 10.1 |
| 28 | | 17.6 | 22.6 | 5.57 | 15.9 |
| 29 | | 20.4 | 10.1 | 3.71 | 17.5 |

SC Immunoglobulin sample no. 13 was selected as a batch with a high level of procoagulant activity whereas sample no. 7 represents SC Immunoglobulin drug product with a low level of procoagulant activity as determined in the analytical testing. Both lots were compared and the test results are shown in Table 3. Both batches differ in the manufacturing process of fraction II/III (25% precipitate) used as starting intermediate fraction for the further manufacturing process of the respective SC Immunoglobulin drug product. The total amount (100%) of fraction II/III pastes used for both batches was PT adsorbed and about 60% passed the C1 esterase inhibitor adsorption in both cases. However, 100% of fraction II/III used for sample no. 7 was AT III adsorbed whereas only an insignificant amount of fraction II/III passed the AT III adsorption step (1.8 %) which was subsequently manufactured into lot no. 13.

The data demonstrate that drug product with a high AT III adsorption rate in the process (sample no. 7) contains very low levels of activated clotting factors and proteolytic activity in the drug product (see Table 3) in comparison to a product manufactured with very little AT III adsorption (sample no. 13).

The method used for the determination of proteolytic activity in SC Immunoglobulin drug product was performed by applying chromogenic substrates (S-2765, S-2238, S-2251 and S-2288) and indicated a significantly lower effect in the drug product if an AT IIIAT III adsorption step was subsequently performed. Due to a relatively low reaction by using substrate S-2251, the presence of plasmin seems to be less relevant for SC Immunoglobulin drug product. Moreover, an increased depletion of FXI-Ag (factor of 3.2), PK-Ag (factor of 6.6) and FXII-Ag (factor of 1.2) measured by ELISA was determined and correlated to the processed AT III adsorption on a high level. The above data were supported by analytical results of intermediate fractions obtained before and after the AT III adsorption step ("Fraction I supernatant prior to AT III adsorption" vs. "After the AT III adsorption step") as presented in the following table, which shows a significant decrease in FXla-like activity, as well as FXI, FXII and PK antigen content.

**Table 2: Comparison of intermediate fractions prior and after the AT III adsorption step**

| Intermediate fraction | AT III content | FXI-ELISA | FXla-like activity | FXII-ELISA | PK-ELISA |
|---|---|---|---|---|---|
| | [IU/mL] | [µg/mL] | [ng/mL] | [mIU/mL] | [µg/mL] |
| Fraction I supernatant prior to AT III adsorption | 0.7 | 3.4 | 295 | 601 | 6.3 |
| After the AT III adsorption step | 0.1 | 0.3 | <10 | 16 | 4.5 |

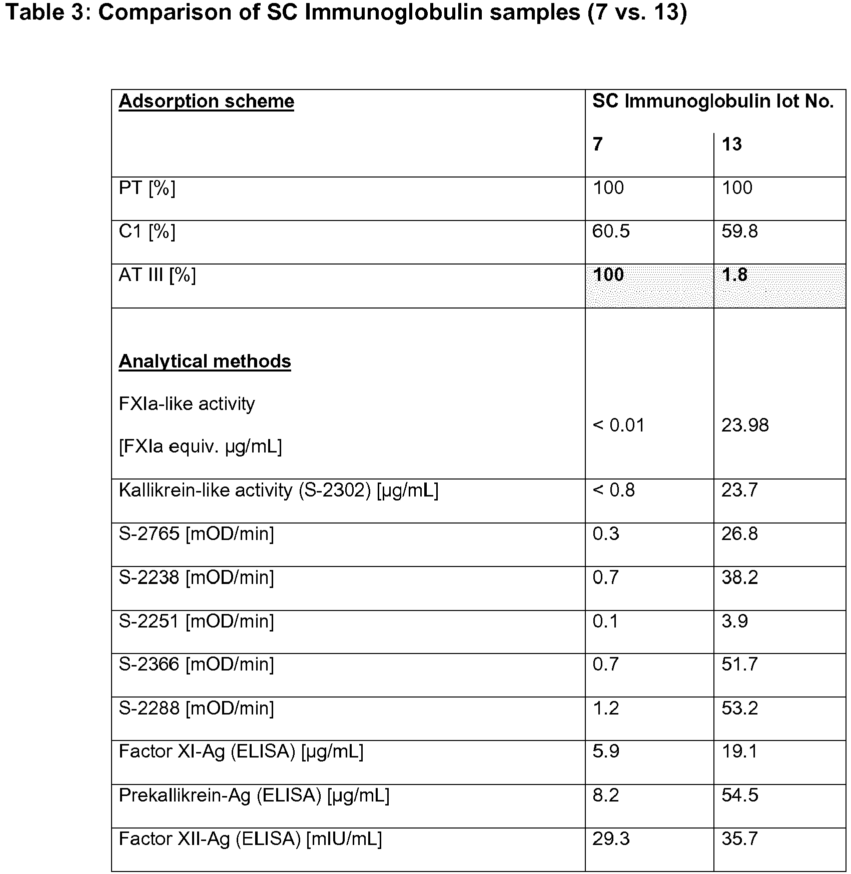

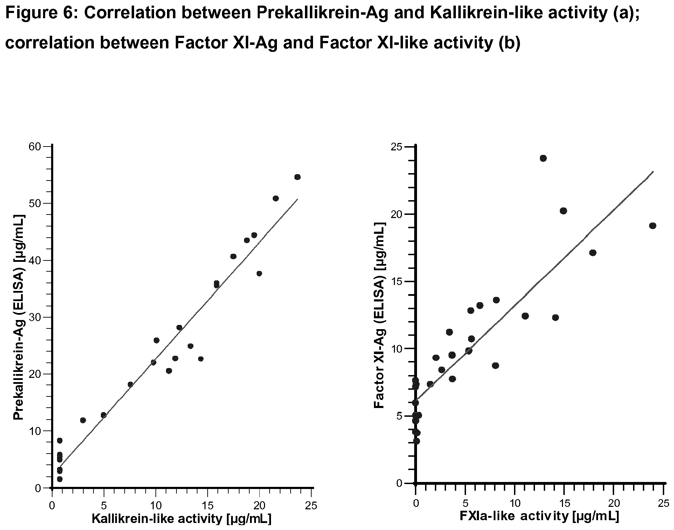

The test results revealed that a high level of AT III adsorption leads to a significant decrease in the procoagulant activity of SC Immunoglobulin drug product. To further detail the effect of the AT III adsorption the content of specific clotting factors in drug product was measured by ELISA. The strong correlation between both prekallikrein-Ag and kallikrein-like activity and FXI-Ag and FXla-like activity is shown in Figure 6.

increasing AT III adsorption led to a depletion of FXI, PK and FXII measured as antigen by ELISA as well as a reduction of FXla- and kallikrein-like activity as shown in Table 2, Table 3 and Figure 5.

The analysis revealed that SC Immunoglobulin lots manufactured with high level of AT III adsorption exhibit low procoagulant activity. These lots reveal lower concentrations of FXI-like activity (in FXI-depleted plasma) as well as a lower kallikrein-like activity values (PKA blank value). The determination of proteolytic activity in SC Immunoglobulin drug product via applying various chromogenic substrates (S-2765, S-2238, S-2251 and S-2288) indicated a significantly lower proteolytic activity in the drug product when AT III adsorption level is high.

increasing AT III adsorption led to a depletion of FXI, PK and FXII measured as antigen by ELISA as well as a reduction of FXla- and kallikrein-like activity as shown in Figure 5.

A strong correlation between both prekallikrein-Ag and kallikrein-Ag and FXI-Ag and FXla-like activity was shown. It was shown that procoagulant activity detected in SC Immunoglobulin is mainly caused by the content of kallikrein and FXla. The data generated within this study provide strong evidence that a high level of AT III adsorption leads to a significant decrease in the procoagulant activity of SC Immunoglobulin drug product.

### Example 2:

Analysis revealed that increasing AT III adsorption during processing of subcutaneous immunoglobulins leads to a depletion of FXI, prekallikrein and FXII antigens as well as a reduction of FXla and kallikrein-like activity. A strong correlation between both kallikrein-antigen and FXI-antigen and FXla-like activity was shown. FXla and Kallikrein were identified as relevant impurities. Based on the finding of procoagulant activity the production process was adapted to include maximum adsorption - that is essentially 100% of the plasma fraction is exposed to the heparin affinity resin.

The removal of AT III from product intermediates for reduction of activated factors activation appears initially paradoxical, because AT III is known to inhibit activated coagulation factors. In fact, ATIII inhibits to a certain extent activated coagulation factors. Further, it is known that heparin accelerates the activity of ATIII by a factor of 1000 (Rosenberg RD: Role of heparin and heparin-like molecules in thrombosis and atherosclerosis. Fed Proc. 1985; 44(2), 404-9). Therefore, the following analysis was performed. In the first experiment, a drug product known to contain FXla and kallikrein-like activities was measured by NaPPT, FXla-like activity and by reactivity towards chromogenic substrate (S2302) (kallikrein-like activity). Then the drug product was treated with 2 U/mL ATIII or with 2 U/mL ATIII plus 10 U/mL heparin. Clotting parameters were determined again (Table 4).

**Table 4: Depletion of activated coagulation factors by AT III and ATIII/heparin**

| Sample description | NaPTT (sec) | FXla-like activity | Chromogenic substrate |
|---|---|---|---|
| | | FXla equiv. (µg/mL) | (S-2302) (mOD/min) |
| Pharmaceutical preparation | 41 | 6.11 | 604 |
| Pharmaceutical preparation + AT III (2U/mL) | 120 | 0.06 | 29 |
| Pharmaceutical preparation + AT III (2U/mL) + heparin (10 U/mL) | No clot formed | < 0.01 | 20 |

While the untreated drug product revealed a shortened NaPTT, 6.11 µg/mL FXla equivalents and elevated reactivity towards S2302 (604 mOD/min), the AT III treated sample displayed a 3-fold prolonged NaPTT, a hundred fold decreased FXla concentration and a 30-fold lesser reactivity towards S2302. When heparin was added, this inhibitory effect was even stronger. There was no clot formed during NaPTT, the FXla content was below detection limit and reactivity towards S2302 was even further reduced.

Those observations are comparable to the situation *In vivo.* The physiological AT III concentration counterbalances the activated coagulation factors up to a certain limit and reaction time, still allowing thrombus formation. Heparin treatment shifts the balance towards anticoagulation and the likelihood of thrombus formation is markedly reduced. Thus the adsorption of AT III to heparin Fractogel is expected to increase the AT III inhibitory capacity and assures that activated factors are inactivated and stable inactive complexes formed. These can then be removed from the plasma fraction by simply removing the heparin affinity resin. This ensures the pharmaceutical preparation will contain essentially no activated serine proteases and will therefore exhibit a reduced adverse event profile.

If however the activated coagulation factors are not removed from the plasma fraction then further processing steps in preparing subcutaneous immunoglobulin preparations will not necessarily lead to the removal of the activated coagulation factors such as FXla. As such it is a requirement that the fractionation process required to prepare the pharmaceutical preparation includes at least one of the plasma fractions to be contacted by heparin or a heparin like substance (eg. heparin affinity resin). Furthermore the use of a heparin affinity resin or similar is advantageous over soluble forms of heparin or heparin like substances as it enables the proteases to be physically be removed from the fraction containing the drug substance. In contrast the addition of ATIII and soluble forms of heparin or heparin like substances will likely lead to complex formation however not necessarily removal as it is possible that given time or subsequent processing steps that the protease/ATIII/heparin complexes may dissociate resulting in the reintroduction of activated serine proteases such as FXIa. Our data indicate that only a removal of the complexes would be effective, instead of an inactivation, and if this removal step is not completed then there is the possibility for proteolytic activity and activated coagulation factor XI to be present in the the final product.

Furthermore it is of note that where AT III is not removed from the plasma fraction by a heparin affinity batch adsorption step that subsequent fractionation steps do nevertheless remove it such that the final subcutaneous immunoglobulin pharmaceutical product is essentially free of AT III. This provides the possibility of a pharmaceutical preparation containing activated proteases but no ATIII and hence accentuates the possibility of adverse events in such products.

### Example 3:

This example provides evidence that the use of heparin affinity resins can be added to other intermediate plasma fractions which contain ATIII in order to remove contaminating activated serine proteases such as FXla.

The removal of activated coagulation factors was investigated for the intermediate fraction, cryo-poor plasma at laboratory scale. The heparin affinity resin (0.5g) was incubated with the cryo-poor plasma (19.5 mL) at room temperature for 30 minutes with stirring. Afterwards the resin was separated from the plasma fraction by centrifugation (Heraeus Co., Multifuge 3SR+ at 1700 rpm for 10 minutes at room temperature). The levels of ATIII, total Factor XI antigen (see method described at 1.2.4 above) and Factor Xla-like activity (see method described at 1.2.1 above) were measured in the cryo-poor plasma before and after exposure to the heparin affinity resin (Table 5). The ATIII activity was approximately 1.1 IU/mL in cryo-poor plasma and this was reduced to 0.6 IU/mL after exposure to the heparin affinity resin. The Factor XI (FXI) levels were reduced from 5.5 µg/mL to 0.3 µg/mL whilst activated Factor XI like activity equivalents were reduced from 2.1 µg/mL to below the assay detection limit of < 0.01 µg/mL. These results suggest that the heparin affinity resin adsorption step is effective at treating plasma fractions comprising ATIII such as cryo-poor plasma.

**Table 5: Levels of ATIII, Factor XI antigen and FXla-like activity in cryo-poor plasma before and after exposure to heparin affinity resin**

| **Sample description** | **ATIII content** | **Factor XI-Ag (ELISA)** | **FXla-like activity** |
|---|---|---|---|
| | **[IU/mL]** | **[µg/mL]** | **FXIa equiv. [µg/mL]** |
| Cryo-depleted plasma **prior** to A Till adsorption | 1.1 | 5.5 | 2.1 |
| Cryo-depleted plasma **after** A Till adsorption | 0.6 | 0.3 | < 0.01 |

The study revealed depletion ratios of 10.2 µg FXI antigen per adsorbed IU of ATIII. The total depletion was about 203 µg FXI antigen per gram of resin.

Additionally the study suggests that the heparin affinity resin can remove both FXla and FXI. It is known that FXI molecule contains heparin binding sites and presumably this contributes to the heparin affinity resins ability to remove both the activated and non-activated FXI.

## Claims

1. Method to reduce FXla activity in a pharmaceutical preparation derived from a plasma fraction comprising antithrombin III wherein the plasma fraction has been preadsorbed to an anion exchange (AEX) matrix and the method comprises contacting the plasma fraction with heparin thereby reducing the activity of FXla per ml of the plasma fraction, wherein the heparin which is brought into contact with the plasma fraction is covalently bound to a matrix, and wherein the plasma fraction is an 8% ethanol supernatant I.

2. Method according to claim 1 wherein the AEX matrix preadsorption of the plasma fraction comprises contacting an intermediate of the plasma fraction with the AEX matrix.

3. Method according to claim 2 wherein the intermediate is cryo-poor plasma.

4. Method according to claim 3 wherein the cryo-poor plasma is adsorbed to AEX matrices to isolate proteins of the Prothrombin complex (PT adsorption) and or to absorb c1-esterase inhibitor (C1 adsorption).

5. Method according to any one of claims 1 to 4 wherein the AEX matrix is DEAE or QAE.

6. Method according to any one of claims 1 to 4 wherein the AEX matrix is an anion exchange membrane.

7. Method according to any one of claims 1 to 6 wherein the pharmaceutical preparation is an immunoglobulin preparation.

8. Method according to any one of claims 1 to 6 wherein the pharmaceutical preparation is an albumin preparation.

## Patentansprüche

1. Verfahren zur Verringerung der FXIa-Aktivität in einer pharmazeutischen Zubereitung, die von einer Antithrombin III umfassenden Plasmafraktion abgeleitet ist, wobei die Plasmafraktion an eine Anionenaustauscher(AEX)-Matrix voradsorbiert wurde und das Verfahren In-Kontakt-Bringen der Plasmafraktion mit Heparin umfasst, wodurch die Aktivität von FXIa pro ml Plasmafraktion verringert wird; wobei das Heparin, das mit der Plasmafraktion in Kontakt gebracht wird, kovalent an eine Matrix gebunden ist und wobei es sich bei der Plasmafraktion um einen 8%-Ethanol-Überstand I handelt.

2. Verfahren nach Anspruch 1, wobei die AEX-Matrix-Voradsorption der Plasmafraktion In-Kontakt-Bringen einer Zwischenstufe der Plasmafraktion mit der AEX-Matrix umfasst.

3. Verfahren nach Anspruch 2, wobei es sich bei der Zwischenstufe um kryoarmes Plasma handelt.

4. Verfahren nach Anspruch 3, wobei das kryoarme Plasma an AEX-Matrizes adsorbiert wird, um Proteine des Prothrombin-Komplexes zu isolieren (PT-Adsorption) und oder c1-Esterase-Inhibitor zu absorbieren (C1-Adsorption).

5. Verfahren nach einem der Ansprüche 1 bis 4, wobei es sich bei der AEX-Matrix um DEAE oder QAE handelt.

6. Verfahren nach einem der Ansprüche 1 bis 4, wobei es sich bei der AEX-Matrix um eine Anionenaustauschermembran handelt.

7. Verfahren nach einem der Ansprüche 1 bis 6, wobei es sich bei der pharmazeutischen Zubereitung um eine Immunglobulinzubereitung handelt.

8. Verfahren nach einem der Ansprüche 1 bis 6, wobei es sich bei der pharmazeutischen Zubereitung um eine Albuminzubereitung handelt.

## Revendications

1. Procédé pour réduire une activité de FXIa dans une préparation pharmaceutique issue d'une fraction plasmatique comprenant de l'antithrombine III, la fraction plasmatique ayant été préadsorbée sur une matrice d'échange d'anions (AEX) et le procédé comprenant la mise en contact de la fraction plasmatique avec de l'héparine, réduisant ainsi l'activité de FXIa par ml de la fraction plasmatique, l'héparine qui est mise en contact avec la fraction plasmatique étant liée de manière covalente à une matrice, et la fraction plasmatique étant un surnageant I d'éthanol à 8 %.

2. Procédé selon la revendication 1, la préadsorption de la fraction plasmatique par une matrice AEX comprenant la mise en contact d'un intermédiaire de la fraction plasmatique avec la matrice AEX.

3. Procédé selon la revendication 2, l'intermédiaire étant du plasma cryo-pauvre.

4. Procédé selon la revendication 3, le plasma cryo-pauvre étant adsorbé sur des matrices AEX pour isoler des protéines du complexe de Prothrombine (adsorption PT) et/ou pour absorber un inhibiteur de c1-estérase (adsorption C1).

5. Procédé selon l'une quelconque des revendications 1 à 4, la matrice AEX étant DEAE ou QAE.

6. Procédé selon l'une quelconque des revendications 1 à 4, la matrice AEX étant une membrane d'échange d'anions.

7. Procédé selon l'une quelconque des revendications 1 à 6, la préparation pharmaceutique étant une préparation d'immunoglobuline.

8. Procédé selon l'une quelconque des revendications 1 à 6, la préparation pharmaceutique étant une préparation d'albumine.
